# EUROPÄISCHE PATENTANMELDUNG

(11) **EP 2 338 589 A1**
(43) Veröffentlichungstag der Anmeldung: **29.06.2011**
(21) Anmeldenummer: 09180637.2
(22) Anmeldetag: 23.12.2009
(51) Int. Cl.: B01F 7/16, B01F 13/06, B01F 15/00, G01N 11/00, A61F 2/46, B01F 3/12

(54) **Verfahren und Vorrichtung zum Zubereiten von Massen aus mindestens zwei Komponenten**

(71) Anmelder: Krämer AG Bassersdorf, 8303 Bassersdorf (CH)
(72) Erfinder: Lutz, Peter, CH-8455, Rüdlingen (CH)
(74) Vertreter: AMMANN PATENTANWÄLTE AG BERN

(57) **Zusammenfassung**

Die Anmeldung betrifft ein Verfahren zum Zubereiten von Massen aus mindestens zwei Komponenten, wobei die Komponenten in einer Mischvorrichtung (4) gemischt werden und die Masse mit einem Applikator (3) appliziert wird. In der Mischvorrichtung (2) wird mindestens eine physikalische oder chemische Eigenschaft gemessen und aus der gemessenen Grösse werden voraussichtliche Zustandswerte der Masse zu bestimmten Zeitpunkten bestimmt. Die bestimmten Zustandswerte werden an eine mit dem Applikator verbundene Steuerungs-und/oder Signaleinrichtung (30) übertragen.

## Beschreibung

Die Erfindung betrifft ein Verfahren zum Zubereiten von Massen aus mindestens zwei Komponenten, wobei die Komponenten in einer Mischvorrichtung gemischt werden und die Masse mit einem Applikator appliziert wird, in der Mischvorrichtung mindestens eine physikalische oder chemische Eigenschaft gemessen wird und aus der gemessenen Grösse voraussichtliche Zustandswerte der Masse zu bestimmten Zeitpunkten bestimmt werden.

Der Begriff Massen umfasst im vorliegenden Zusammenhang alle Stoffgemische, die in einem flüssigen oder plastischen Zustand vorliegen. Unter den Begriff fallen insbesondere auch aushärtbare Massen wie beispielsweise Polymere.

Aus der Patentanmeldung EP-1804057-A1 ist ein Verfahren bekannt, bei dem in einem Applikator für Knochenzement die Impedanz gemessen und daraus der voraussichtliche Verlauf der Aushärtung errechnet wird. Dabei kann die Messung erst beginnen, wenn die gemischte Masse in den Applikator gefüllt ist.

Ausgehend von diesem Stand der Technik liegt der Erfindung die Aufgabe zugrunde, ein Verfahren zum Zubereiten von Massen aus mindestens zwei Komponenten vorzuschlagen, bei dem Zustandswerte der Masse vor, während oder nach der Applikation am Applikator zur Verfügung stehen.

Diese Aufgabe wird erfindungsgemäss durch das Verfahren gemäss Patentanspruch 1 gelöst.

Dieses Verfahren hat den Vorteil, dass bereits während des Mischvorganges eine Messreihe zur Verfügung steht, die es erlaubt, den Mischvorgang zu steuern. Die Steuerungs-und/oder Signaleinrichtung am Applikator informiert den Benutzer stets über den aktuellen Zustand der Masse oder verhindert das Austragen der Masse aus dem Applikator, wenn deren aktueller Zustand nicht den Vorgaben entspricht.

Besondere Ausführungsarten des Verfahrens sind in den abhängigen Verfahrensansprüchen umschrieben.

Die Erfindung betrifft auch eine Vorrichtung zum Zubereiten von Massen aus mindestens zwei Komponenten, mit einer Mischvorrichtung, einem Applikator sowie Messmitteln und Auswertemitteln.

Es sind Vorrichtungen mit Messmitteln bekannt, beispielsweise aus dem Dokument WO95/01832A1. Dabei werden aber die gemessene Werte nur zum Steuern des Mischvorgangs verwendet.

Der Erfindung liegt hinsichtlich der Vorrichtung die Aufgabe zugrunde, eine Vorrichtung zum Zubereiten von Massen aus mindestens zwei Komponenten vorzuschlagen, bei der Zustandswerte der Masse vor, während oder nach der Applikation am Applikator zur Verfügung stehen.

Gelöst wird diese Aufgabe erfindungsgemäss dadurch, dass die Messmittel mindestens eine in der Mischvorrichtung angeordnete Sonde aufweisen und dass der Applikator eine Steuerungs-und/oder Signaleinrichtung aufweist.

Diese Vorrichtung hat den Vorteil, dass bereits während des Mischvorganges eine Messreihe zur Verfügung steht, die es erlaubt, den Mischvorgang zu steuern. Die Steuerungs-und/oder Signaleinrichtung am Applikator informiert den Benutzer stets über den aktuellen Zustand der Masse oder verhindert das Austragen der Masse aus dem Applikator, wenn deren aktueller Zustand nicht den Vorgaben entspricht.

Besondere Ausführungsarten der Vorrichtung sind in den abhängigen Vorrichtungsansprüchen umschrieben.

Ausführungsbeispiele der Erfindung werden nachstehend unter Bezugnahme auf die angefügte Figur näher beschrieben.

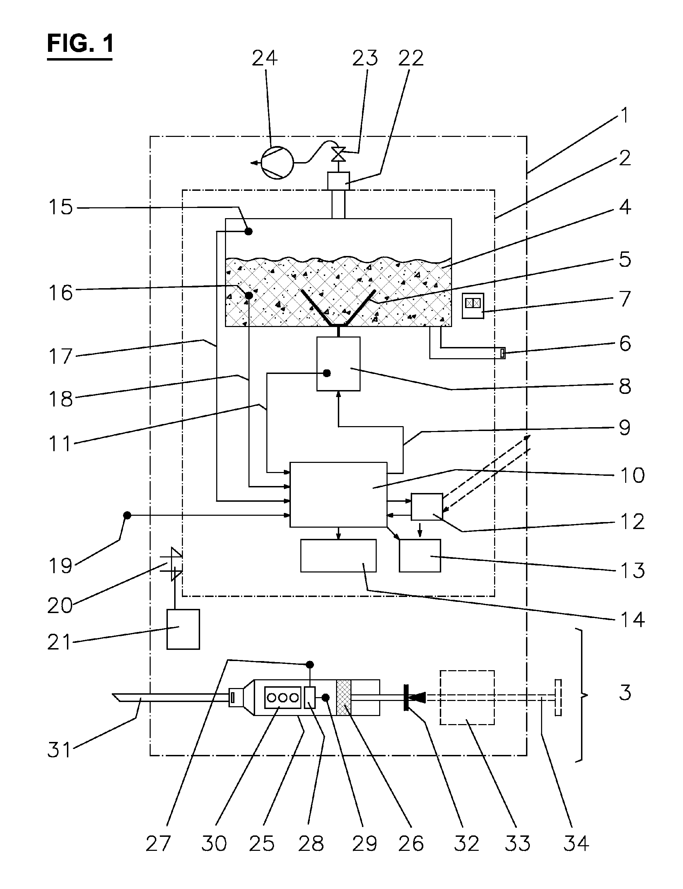

Die einzige Figur 1 zeigt schematisch eine Vorrichtung zum Zubereiten und Applizieren von Massen aus mindestens zwei Komponenten.

Die Vorrichtung ist in der Figur als Ganzes mit der Bezugszahl 1 bezeichnet und dient beispielsweise zum Zubereiten einer aushärtbaren Masse, beispielsweise Knochenzement. Sie besteht aus einer Mischvorrichtung 2 und einem Applikator 3. Mit der die beiden genannten Elemente umgebenden strichpunktierten Linie ist angedeutet, dass die Mischvorrichtung 2 und der Applikator 3 auch in einem einzigen Gehäuse zusammengefasst sein können. Eine Mischkammer 4 ist zur Aufnahme der zu mischenden Komponenten vorgesehen. Sie ist luftdicht verschliessbar und über einen Vakuumanschluss 22 evakuierbar. Letzterer ist durch ein Vakuumventil 23 verschliessbar. Eine Vakuumpumpe 24 kann innerhalb oder ausserhalb der Vorrichtung 1 vorgesehen sein. In der Mischkammer 4 befindet sich ein Mischelement 5, das durch einen Mischantrieb 8 antreibbar ist. Mit 6 ist ein Auslass bezeichnet, der durch eine Auslassaktivierung 7 betätigbar ist. Die Vorrichtung 1 ist mit einem Energieanschluss 20 und optional mit einer Energiequelle 21, beispielsweise in Form eines Akkumulators versehen.

Durch Inbetriebnahme des Mischantriebs 8 werden die zu mischenden Komponenten in der Mischkammer 4 gründlich durchmischt. Dabei werden über einen ersten Sensor 15 physikalische und/oder chemische Daten in der Atmosphäre der Mischkammer 4 erfasst und über eine Signalleitung 17 einem Prozessor 10 zugeleitet. Die genannten Daten können beispielsweise den Druck, die Temperatur oder den Gehalt einer bestimmten Verbindung in der Mischkammer 4 betreffen. Ein weiterer Sensor 16 erfasst physikalische und/oder chemische Daten der Masse und leitet die Messwerte über eine Signalleitung 18 dem Prozessor 10 zu. Die genannten Daten können beispielsweise die Dichte, die Temperatur, die Viskosität oder den Gehalt eines bestimmten Stoffes in der Masse betreffen. Über einen weiteren Sensor 19 können auch Daten aus der Umgebung der Vorrichtung 1 erfasst und an den Prozessor geleitet werden. Diese Daten können beispielsweise die Luftfeuchtigkeit, den Luftdruck oder die Temperatur betreffen. Schliesslich erhält der Prozessor 10 auch Daten vom Mischantrieb 8, wie dies durch die Signalleitung 11 angedeutet ist. Beispielsweise kann das Drehmoment des Mischantriebs 8 über einen entsprechenden Sensor gemessen werden oder der Drehmomentverlauf wird über die Änderung der Stromaufnahme des Mischantriebs 8 verfolgt. Aus der Zunahme des Drehmoments kann auf eine Zunahme der Viskosität der Masse geschlossen werden. Selbstverständlich kann die Viskosität und deren Änderung auch anders bestimmt werden, beispielsweise durch eine Impedanzmessung. Anstelle der Signalleitungen 11, 17 und 18 können auch drahtlose Mittel zur Übertragung der Signale verwendet werden.

Mittels der erhaltenen Daten errechnet der Prozessor 10 den Zustand des Gemischs. Der Prozessor 10 kann den Mischantrieb 8 über eine Steuerleitung 9, die Vakuumpumpe 24 oder das Vakuumpumpenventil 23 je nach Zustand des Gemischs steuern. Weiter kann der Prozessor 10 mit einer Signaleinrichtung 14 verbunden sein, welche eine Bedienperson beispielsweise über den momentanen Zustand des Gemischs und/oder über den empfohlenen Zeitbereich für die Applikation der Masse informieren. Sobald die gewünschte Mischqualität erreicht ist, wird der Mischantrieb 8 automatisch ausgeschaltet.

Während und nach dem Mischvorgang errechnet der Prozessor 10 den unter den gemessenen Parametern zu erwartenden Verlauf der Aushärtung der Masse. Der errechnete Aushärtungsverlauf wird an eine Datenübertragungseinheit 12 per Kabel oder kabellos weitergeleitet. Die Datenübertragungseinheit 12 kann in den Prozessor 10 integriert sein. Ebenso kann dem Prozessor 10 ein Datenspeicher 13 zugeordnet oder in diesen integriert sein.

Nach erfolgtem Mischen wird die Masse in einen Applikator 3 transferiert. Dies kann manuell oder per Antrieb bzw. dosiert erfolgen. Hierzu wird vorgängig der Applikator 3 mit dem Auslass 6 der Mischvorrichtung 2 verbunden. Die am Applikator 3 angeordnete Signaleinrichtung 30 kann eine einfache Anzeigeeinrichtung sein, die beispielsweise mit Hilfe von Leuchtdioden anzeigt, ob die Masse appliziert werden kann oder nicht. Ähnlich wie bei einer Verkehrsampel das orange Licht kann auch ein Anzeigeelement vorhanden sein, das einen kurz bevorstehenden Wechsel ankündigt. Die Signaleinrichtung 30 kann aber auch akustische oder tastbare Signalmittel beinhalten. Der Applikator 3 besteht im dargestellten Beispiel aus einem Zylinder 25 zur Aufnahme der gemischten Masse und einem Kolben 26 zum Ausstossen der Masse durch eine Applikatorkanüle 31. Der Kolben 26 kann über ein Betätigungsglied 32 manuell oder über einen Applikatorantrieb 33 motorisch im Zylinder 25 vorgeschoben werden. Wenn die Mischvorrichtung 2 und der Applikator 3 in einem gemeinsamen Gehäuse 1 untergebracht sind, kann ein aus dem Gehäuse nach aussen ragendes Betätigungsglied 34 vorgesehen sein.

Vor, während oder nach dem Transfer der Masse in den Applikator 3 wird der errechnete Aushärtungsverlauf mittels der Datenübertragungseinheit 12 an eine entsprechende Datenübertragungseinheit 28 im Applikator 3 übertragen und laufend angepasst; dies kann per Kabel oder kabellos erfolgen. Signaleinrichtungen 14, 30 an der Mischvorrichtung 2 bzw. am Applikator 3 geben dem Anwender Auskunft über den aktuellen Zustand der Masse. Die Signaleinrichtung 30 im Applikator 3 ist mit der im Applikator 3 integrierten Datenübertragungseinheit 28 verbunden oder direkt Bestandteil davon. Über die reine Signalisierung hinaus kann das Austragen der Masse aus dem Applikator blockiert werden, wenn sich die festgestellten Parameter der Masse noch nicht oder nicht mehr in einem vorgeschriebenen Bereich befinden. Der Applikator 3 kann durch Fremdspeisung versorgt werden oder energetisch autark sein.

So lange nach dem Transfer der Masse in den Applikator 3 in der Mischkammer 4 noch Masse zurückbleibt, werden die genannten Parameter in der Mischkammer 4 weiter gemessen und der Verlauf bei Abweichung korrigiert. Dadurch ist es möglich, mit der Menge der in der Mischkammer 4 vorhandenen Masse den Applikator 3 mehrmals zu befüllen. Auch in diesem Fall stehen am Applikator 3 stets aktuelle Zustandswerte der Masse zur Verfügung.

Im dargestellten Beispiel sind auch am Applikator 3 Sensoren angeordnet, nämlich ein Umgebungssensor 27 und ein Sensor 29 zur Erfassung der Eigenschaften der Masse. Diese Sensoren 27, 29 nehmen kontinuierlich oder schrittweise physikalische und/oder chemische Parameter aus dem Gemisch sowie Fremdeinwirkungen aus der Umgebung auf den Applikator auf. Diese Daten werden kontinuierlich oder schrittweise durch die genannte Datenübertragungseinheit 28 per Kabel oder kabellos an die Datenübertragungseinheit 12 und von dieser an den Prozessor 10 der Mischvorrichtung 2 gesendet. Der Prozessor 10 bewertet die neu erfassten Daten und passt im Falle einer Abweichung zum vorher errechneten Aushärtungsvorgang den weiter zu erwartenden Aushärtungsverlauf an, wobei der gegebenenfalls korrigierte Verlauf wiederum in der vorher beschriebenen Weise an den Applikator 3 übermittelt wird. Das Erfassen der Daten am Applikator 3 sowie deren Kommunikation mit dem Prozessor 10 kann so lange erfolgen bis die Applikation beendet oder eine weitere Applikation unmöglich ist. Die während der gesamten Anwendung d.h. vom Mischvorgang bis zum Ende der Applikation erfassten Daten können auf geeignete Weise gespeichert und zu einem späteren Zeitpunkt herausgelesen und beispielsweise für die Qualitätskontrolle verwendet werden.

Die vorangehend beschriebene Vorrichtung 1 kann auch als All-in-One-Gerät bzw. Handgerät ausgeführt sein, in dem die Mischvorrichtung 2 und der Applikator 3 integriert sind und in dem somit der Applikator 3 nicht zum Transfer des Gemischs an die Mischvorrichtung 2 angeschlossen werden muss. Das Konzept des Mischens der Komponenten sowie das Feedback der Mischqualität kann auf dieselbe Weise erfolgen wie vorangehend beschrieben. Aus den erfassten physikalischen und/oder chemischen Daten wird ebenfalls ein Zustands- bzw. Aushärtungsverlauf errechnet. Wiederum kann dieser Verlauf durch gemessene Eingangsgrössen kontinuierlich oder schrittweise korrigiert werden. Die direkte oder indirekte Kommunikation zwischen der Datenübertragungseinheit 28 am Applikator 3 und der Datenübertragungseinheit 12 in der Mischvorrichtung 2 kann per Kabel oder kabellos erfolgen. Das All-in-One-Gerät kann durch Fremdspeisung versorgt werden oder energetisch autark sein.

### Bezugszeichenliste

- 1: Vorrichtung
- 2: Mischvorrichtung
- 3: Applikator
- 4: Mischkammer
- 5: Mischelement
- 6: Auslass
- 7: Auslassaktivierung
- 8: Mischantrieb
- 9: Steuerleitung
- 10: Prozessor
- 11: Signalleitung
- 12: Datenübertr.- Einheit
- 13: Datenspeicher
- 14: Signaleinrichtung
- 15: Sensor Atm. Mischkammer
- 16: Sensor Masse Mischk.
- 17: Signalleitung
- 18: Signalleitung
- 19: Umgebungssensor
- 20: Energieanschluss
- 21: Energiequelle
- 22: Vakuumanschluss
- 23: Vakuumventil
- 24: Vakuumpumpe
- 25: Zylinder
- 26: Kolben
- 27: Sensor Applikator
- 28: Datenübertragungseinh.
- 29: Sensor Masse Applikator
- 30: Signaleinrichtung

- 31: Applikatorkanüle
- 32: Betätigungsglied
- 33: Applikatorantrieb
- 34: Betätigungsglied
- 35:
- 36:
- 37:
- 38:
- 39:
- 40:
- 41:
- 42:
- 43:
- 44:
- 45:
- 46:
- 47:
- 48:
- 49:
- 50:
- 51:
- 52:
- 53:
- 54:
- 55:
- 56:
- 57:
- 58:
- 59:
- 60:

## Patentansprüche

1. Verfahren zum Zubereiten von Massen aus mindestens zwei Komponenten, wobei die Komponenten in einer Mischvorrichtung (2) gemischt werden und die Masse mit einem Applikator (3) appliziert wird, in der Mischvorrichtung (2) mindestens eine physikalische oder chemische Eigenschaft gemessen wird und aus der gemessenen Grösse voraussichtliche Zustandswerte der Masse zu bestimmten Zeitpunkten bestimmt werden, **dadurch gekennzeichnet, dass** die bestimmten Zustandswerte an eine am Applikator (3) angeordnete Steuerungs-und/oder Signaleinrichtung (30) übertragen werden.

2. Verfahren nach Anspruch 1, **dadurch gekennzeichnet, dass** die Bestimmung der voraussichtlichen Zustandswerte durch Berechnung und/oder durch Abrufen gespeicherter Werte erfolgt.

3. Verfahren nach einem der vorangehenden Ansprüche, **dadurch gekennzeichnet, dass** im Applikator (3) mindestens eine physikalische oder chemische Eigenschaft gemessen und das Ergebnis an die Mischvorrichtung (2) übertragen wird, wo ein Ist-Zustandswert der Masse bestimmt und gegebenenfalls die genannten voraussichtlichen Zustandswerte korrigiert werden.

4. Verfahren nach einem der vorangehenden Ansprüche, **dadurch gekennzeichnet, dass** die bestimmten Zustandswerte auch an eine in der Mischvorrichtung (2) angeordnete Signaleinrichtung (14) übermittelt werden.

5. Vorrichtung (1) zum Zubereiten von Massen aus mindestens zwei Komponenten, mit einer Mischvorrichtung (2), einem Applikator (3) sowie Messmitteln und Auswertemitteln (10, 13), **dadurch gekennzeichnet, dass** die Messmittel mindestens eine mit der Mischvorrichtung (2) verbundene Sonde (15, 16, 19) aufweisen und dass der Applikator (3) eine Steuerungs-und/oder Signaleinrichtung (30) aufweist.

6. Vorrichtung nach Anspruch 5, **dadurch gekennzeichnet, dass** die Auswertemittel einen Prozessor (10) enthalten.

7. Vorrichtung nach einem der Ansprüche 5 bis 6, **dadurch gekennzeichnet, dass** die Auswertemittel einen Datenspeicher (13) enthalten.

8. Vorrichtung nach einem der Ansprüche 5 bis 7, **dadurch gekennzeichnet, dass** die Steuerungs-und/oder Signaleinrichtung (30) eine optische Anzeigevorrichtung enthält.

9. Vorrichtung nach einem der Ansprüche 5 bis 8, **dadurch gekennzeichnet, dass** die Steuerungs-und/oder Signaleinrichtung (30) eine akustische Signaleinrichtung enthält.

10. Vorrichtung nach einem der Ansprüche 5 bis 9, **dadurch gekennzeichnet, dass** die Steuerungs-und/oder Signaleinrichtung (30) eine haptische Signaleinrichtung enthält.

11. Vorrichtung nach einem der Ansprüche 5 bis 10, **dadurch gekennzeichnet, dass** die Steuerungs-und/oder Signaleinrichtung (30) Mittel zum Sperren der Austragsfunktion des Applikators (3) aufweist.

12. Vorrichtung nach einem der Ansprüche 5 bis 11, **dadurch gekennzeichnet, dass** der Applikator (3) mindestens eine Sonde (27, 29) und Mittel (28) zum Übertragen deren Signale an die Mischvorrichtung (2) aufweist.

13. Vorrichtung nach einem der Ansprüche 5 bis 12, **dadurch gekennzeichnet, dass** die Mischvorrichtung eine evakuierbare Mischkammer (4) aufweist.

14. Vorrichtung nach einem der Ansprüche 5 bis 13, **dadurch gekennzeichnet, dass** die Mischvorrichtung (2) eine Signaleinrichtung für die bestimmten Zustandswerte aufweist.
